# EUROPÄISCHE PATENTANMELDUNG

(11) **EP 1 882 579 A1**
(43) Veröffentlichungstag der Anmeldung: **30.01.2008**
(21) Anmeldenummer: 07013663.5
(22) Anmeldetag: 12.07.2007
(51) Int. Cl.: B29C 65/82, G01N 25/72

(54) **Verfahren und Vorrichtung zur Herstellung von mit einer Siegelnaht versehenen Gegenständen**

(30) Priorität: 27.07.2006 CH 12212006
(71) Anmelder: Soudronic AG, 8962 Bergdietikon (CH)
(72) Erfinder: Oberholzer, Marcel, 8963 Kindhausen (CH); Gysi, Peter, 5454 Bellikon (CH)
(74) Vertreter: Schalch, Rainer

(57) **Zusammenfassung**

Bei der Herstellung von Deckeln mit Abreissfolie wird deren Siegelnaht in der Siegelstation oder nachfolgend durch ein Wärmebild der Siegelnaht auf korrekte Versiegelung geprüft. Dazu ist bei der Siegelstation (40) eine Einrichtung (46) vorgesehen, die eine Wärmeabbildung der noch heissen Siegelnaht vornimmt.

## Beschreibung

Die Erfindung betrifft ein Verfahren zur Herstellung einer Folge von Gegenständen, insbesondere Deckeln mit einer Abreissfolie, mit einer Siegelnaht. Ferner betrifft die Erfindung eine Vorrichtung zur Herstellung einer Folge von Gegenständen mit einer Siegelnaht, insbesondere von Deckeln mit einer Abreissfolie.

### Stand der Technik

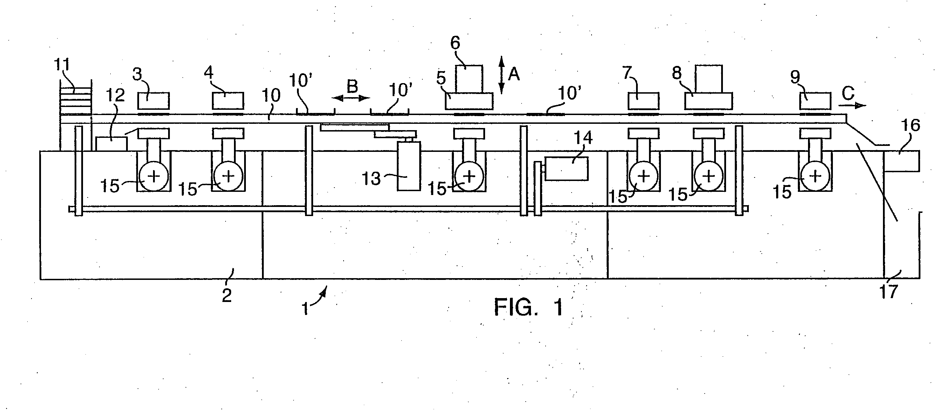

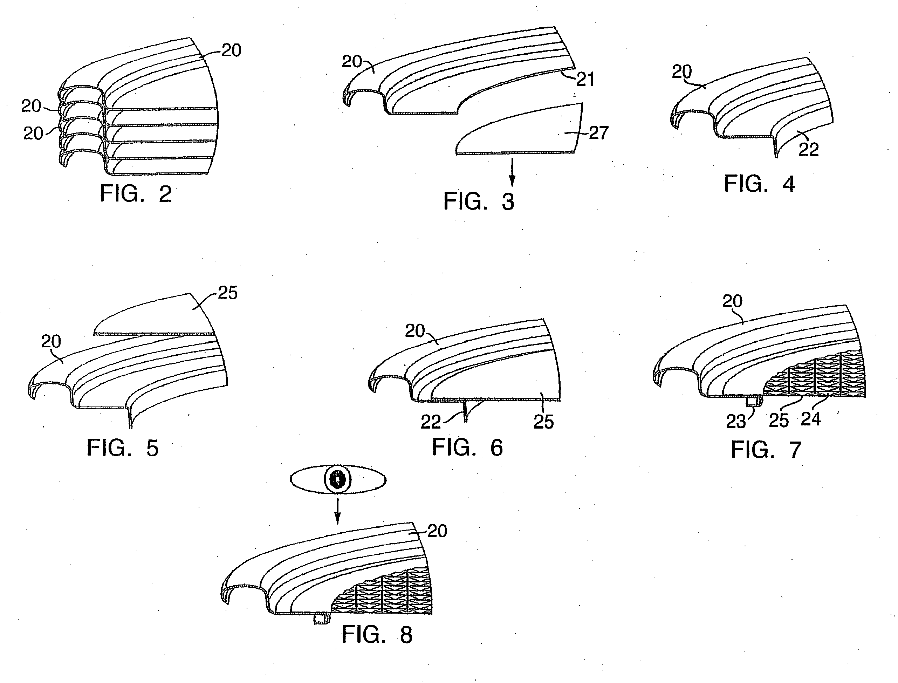

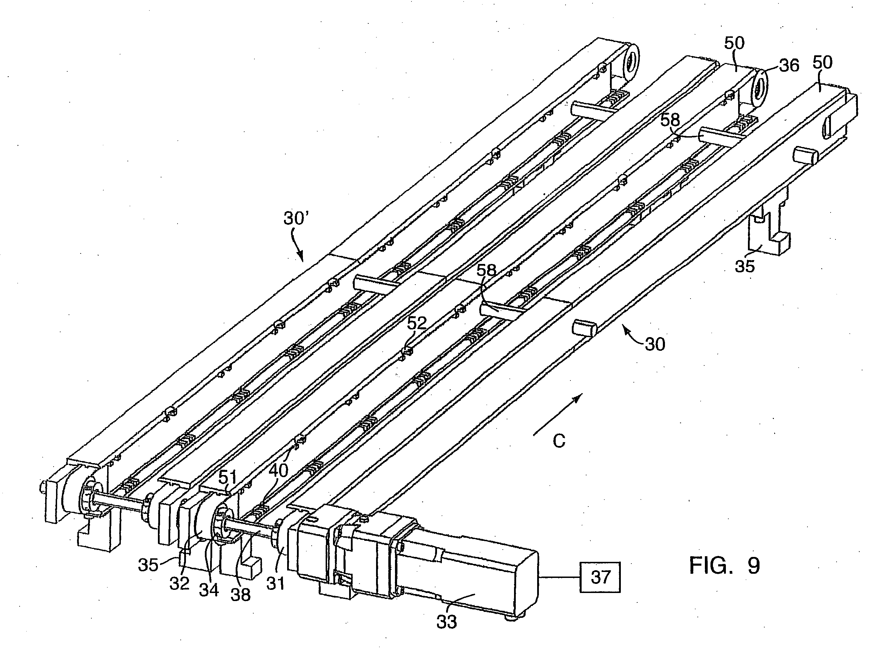

Es ist bekannt, Deckel für dosen- oder büchsenartige Verpackungen als auf der Verpackung oberseitig permanent befestigte Metalldeckel auszuführen, die eine Entnahmeöffnung ausbilden, die bis zum ersten Gebrauch des Verpackungsinhaltes durch eine durch Heisssiegelung auf dem Deckel aufgebrachte, abreissbare Folie, insbesondere Metallfolie, verschlossen ist. Ein zusätzlicher, über dem Metalldeckel angeordneter Deckel aus Kunststoff macht die Verpackung während der Verbrauchsdauer für deren Inhalt wiederverschliessbar. Bekannte Verfahren bzw. Vorrichtungen zur Herstellung solcher Metalldeckel werden nachfolgend anhand der Figuren 1 bzw. 9 näher erläutert. Die Figuren 2 bis 8 dienen zur Erläuterung von Herstellungsschritten bei der Herstellung solcher Deckel.

Figur 1 zeigt dabei eine schematische Seitenansicht einer solchen Vorrichtung 1, welche auf einem Maschinengestell 2 mehrere Bearbeitungsstationen 3 bis 9 aufweist. Eine Fördereinrichtung 10, 13, 14 fördert Gegenstände in Förderrichtung, welche durch den Pfeil C angedeutet ist, vom Anfang der Vorrichtung beim Stapel 11 bis zum Ende der Vorrichtung, wo die Gegenstände über Rutschen in die Ablagen 16 oder 17 gelangen. Vom Stapel 11 werden die Gegenstände auf bekannte Weise abgestapelt und gelangen in die Förderanordnung. Diese weist zwei jeweils einzeln seitlich der Gegenstände angeordnete lange Schienen 10 auf, welche die auf Ablagen 10' bzw. in den Stationen 3 bis 9 liegenden Gegenstände beim Anheben der Stangen 10 mittels des Antriebes 14 in Richtung A nach oben anheben und sie danach durch eine Vorwärtsbewegung in Richtung des Pfeiles B (gleichgerichtet wie der Pfeil C) durch den Kurbelantrieb 13 um einen Betrag nach vorne versetzen. Danach werden die Stangen in Richtung des Pfeiles A nach unten bewegt wobei die Gegenstände wiederum auf ihren Ablagestellen abgelegt werden. Die Stangen 10 werden danach unterhalb der Gegenstandsablagepositionen in Pfeilrichtung B entgegen dem Pfeil C nach hinten bewegt um danach den beschriebenen Vorgang erneut durchzuführen. Die Gegenstände ruhen zwischen dem Transport auf ihren Ablagepositionen bzw. befinden sich in den Bearbeitungsstationen und werden dort bearbeitet. Nach einem Bearbeitungsschritt aller Bearbeitungsstationen erfolgt die erneute Förderung. Figur 2 zeigt gestapelte metallene Deckelrohlinge 20 als Beispiel für Gegenstände, wie sie im Stapel 11 vorliegen. Diese Rohlinge 20 sind z.B. runde Metallscheiben von z.B. 11 cm Durchmesser. Natürlich sind andere Grundformen, z.B. quadratische oder rechteckige Scheiben und andere Durchmesser ohne weiteres möglich. Die Rohlinge 20 sind bereits in einer nicht dargestellten Bearbeitungsmaschine an ihrem Rand wie in Figur 2 gezeigt vorgeformt. In der Figur 2 und den nachfolgenden Figuren ist jeweils nur ein Sektor der ganzen Scheibe dargestellt, um die Zeichnungen zu vereinfachen. In der ersten Bearbeitungsstation 3 von Figur 1 wird durch eine Stanzbearbeitung mit Ober- und Unterwerkzeug eine Öffnung in die Scheibe gestanzt, was in Figur 2 ersichtlich ist, in welcher der Rand der Öffnung mit 21 bezeichnet ist und die ausgestanzte runde Scheibe mit 27. Diese Scheibe gelangt als Abfall in den Behälter 12 von Figur 1. Die Stanzberarbeitungsstation 3 wird - wie dies auch bei den weiteren Stationen der Fall ist - durch einen Antrieb 15 angetrieben. Bei der Bearbeitungsstation 4 erfolgt ein Ziehen des Randes 21 nach unten, wodurch der in Figur 3 gezeigte Verlauf 22 des Randes erzielt wird. Die ringförmigen Deckelrohlinge 20 gelangen nun in die Bearbeitungsstation 5, in welcher eine Folie 25 über der Öffnung des Deckels 20 plaziert und dort durch Heissversiegelung befestigt wird, was in den Figuren 5 und 6 ersichtlich ist. Die Metallfolie 25 ist dazu auf bekannte Weise an ihrer Unterseite mit einer Kunststoffschicht versehen. Der benötigte runde Folienzuschnitt 25 wird in der Regel in der Station 5 aus einer breiten Folienbahn ausgestanzt und über der Mittelausnehmung der ringförmigen Scheibe plaziert und durch die Heissversiegelungsstation wird die Folie unter Hitzeeinwirkung am Rand der runden Ausnehmung des Teils 20 unter Hitzeinwirkung angepresst, so dass die Folie 25 mit dem metallenen Deckel 20 durch Aufschmelzen und nachfolgendes Abkühlen der Kunststoffschicht dicht verbunden wird. Dies ist bekannt und wird hier nicht näher erläutert. Zur Abkühlung kann allenfalls eine Kühlbearbeitungsstation 7 vorgesehen sein. In der Bearbeitungsstation 8 wird die Folie 25 mit einer Prägung 24 (Figur 7) versehen, und es wird weiter der Rand 22 zum fertigen Rand 23 umgebördelt. In einer ebenfalls als Bearbeitungsstation zu bezeichnenden Prüfstation 9 nach Stand der Technik werden die nun fertigen Deckel einer Prüfung unterzogen, welche in der Regel eine Dichteprüfung für die auf dem Deckel aufgebrachte Abziehfolie 25 umfasst, wozu eine Luftdruckdifferenz zwischen Unterseite und Oberseite des Deckels erzeugt wird. Ist die Folie dicht auf dem restlichen Metalldeckel befestigt, so gelangt der Deckel in die Aufnahme 16 für die fertigen Deckel. Wird eine Undichtigkeit festgestellt, so gelangt der Deckel über die andere dargestellte Rutsche in den Abfallbehälter 17.

Figur 9 zeigt in schaubildlicher Darstellung eine weitere bekannte (WO 2006/017953) Vorrichtung mit einer Fördereinrichtung 30, die zwei Zahnriemen 31 und 32 aufweist, welche insbesondere mit ihren Oberflächen in den selben Ebenen liegen, also koplanar verlaufen, und welche am Anfang und am Ende der Fördervorrichtung über Umlenkrollen 34, 36 geführt sind, so dass sich ein endloser Zahnriemenantrieb in der für die Anzahl der Bearbeitungsstationen notwendigen Länge ergibt. Die schrittweise, mit den Bearbeitungsstationen synchronisierte Zahnriemenbewegung wird durch einen Schrittmotor oder Servomotor bewirkt, welcher die Zahnriemen durch Zahnrollen antreibt, wie dies in der Figur mit dem Motor 33 und der Antriebsachse 38 ersichtlich ist. Falls weitere Fördervorrichtungen, wie die Fördervorrichtung 30' vorgesehen sind, so können deren Zahnriemen über denselben Motor über weitere Antriebsachsen angetrieben werden oder über einen eigenen motorischen Antrieb verfügen. Der Motor 33 wird durch eine Steuerung 37 zur Vornahme der schrittweisen Vorwärtsbewegung der Zahnriemen gesteuert, welche Steuerung 37 entweder eine gesamte Steuerung der Deckelherstellungsvorrichtung ist, welche auch die Bearbeitungsstationen steuert, oder welche Steuerung 37 eine Steuerung nur für die Fördervorrichtung ist, welche mit einer übergeordneten Steuerung für die Deckelherstellungsvorrichtung kommuniziert. Diese Fördervorrichtung 30 ist zur Förderung von deckelförmigen Gegenständen der geschilderten Art ausgestaltet und dient wiederum zum taktweisen Fördern der Gegenstände zur einzelnen Bearbeitungsstationen, welche vorzugsweise die vorgängig geschilderten Bearbeitungsstationen zur Deckelherstellung sind. Diese Bearbeitungsstationen sind in Figur 9 nicht gezeigt, es ist dem Fachmann aber klar, wie er diese entlang der Fördervorrichtung anordnen kann, um die jeweilige Bearbeitung durchzuführen. Mit der dargestellten Fördereinrichtung 30 können die Gegenstände bzw. Deckel mit hoher Taktzahl von z.B. 200 Gegenständen pro Minute und mit reproduzierbaren Teilschritten zwischen den Bearbeitungsstationen gefördert werden.

Bei einem Herstellungstakt von ca. 200 Deckeln pro Minute steht für die erwähnte Qualitätsüberwachung mit Druckdifferenzüberwachung nur noch eine sehr kurze Zeit zur Verfügung.

### Darstellung der Erfindung

Der Erfindung liegt die Aufgabe zu Grunde, hier eine Verbesserung zu schaffen.

Zur Lösung der Aufgabe wird bei dem eingangs genannten Verfahren in der Siegelstation oder in einer an die Siegelstation anschliessenden Station ein Wärmeabbild der Siegelnaht aufgenommen und ausgewertet. Ferner wird die Aufgabe mit der Herstellungsvorrichtung nach Anspruch 8 gelöst.

Dadurch, dass in der Siegelstation, oder allenfalls in einer anschliessenden Station, in der der Gegenstand noch eine heisse Siegelnaht aufweist, ein Abbild des Wärmeverlaufs der Siegelnaht aufgenommen und ausgewertet wird, kann auf einfache und rasche Weise festgestellt werden, ob die Siegelung korrekt erfolgt ist und damit die Siegelnaht dicht ist. Damit kann die Druckprüfung entfallen. Eine korrekt gesiegelte Siegelnaht weist einen weitgehend einheitlichen Temperaturverlauf entlang ihrer Länge auf, der sich in der Abbildung entsprechend einheitlich darstellt. Zu kalte oder zu heisse Stellen weisen dahingegen im Abbild entsprechend abweichendes Aussehen auf und können als potentiell fehlerhafte Stellen erkannt werden, worauf der Gegenstand als fehlerhaft ausgeschieden werden kann.

Die Aufnahme des Abbildes, sei dies als eigentliches Bild oder als Temperaturverlauf, erfolgt bevorzugt in der Siegelstation selber und bevorzugt direkt nach dem Öffnen des Siegelwerkzeuges nach erfolgter Siegelung. Es können als Lichtleiter z.B. Glasfasern als Aufnehmer für die Wärmestrahlung entlang der Siegelnaht vorgesehen sein, deren Ausgang zusammengefasst angeordnet von einer infrarotempfindlichen Kamera aufgenommen werden kann, damit ein einziges Bild der gesamten Siegelnaht entsteht. Bei einer anderen Ausführung werden mehrere Bilder aufgenommen. Es können auch einzelne infrarotempfindliche Sensoren oder Thermometer so angeordnet sein, dass sie ein Wärmeabbild der Siegelnaht erfassen, das Fehlstellen erkennen lässt. Bei einer anderen Ausführungsform wird das Bild mittels durch eine Siegelscheibe hindurch geführte Lichtleiter, insbesondere Glasfasern, aufgenommen.

### Kurze Beschreibung der Zeichnungen

Im Folgenden wird der Stand der Technik und werden Ausführungsbeispiele der Erfindung anhand der Zeichnungen näher erläutert. Dabei zeigt
Figur 1 eine schematische Seitenansicht einer Vorrichtung nach Stand der Technik;
Figur 2 bis 8 Sektoren von Metalldeckeln zur Erläuterung von deren Herstellung;
Figur 9 eine weitere Vorrichtung nach Stand der Technik;
Figur 10 ein erstes Ausführungsbeispiel einer Siegelstation der Vorrichtung zur Herstellung von Gegenständen gemäss der Erfindung;
Figur 11 eine Teilansicht der Siegelstation von Figur 10;
Figur 12 eine schematische Ansicht der Glasfaserführung;
Figur 13 eine weitere Ausführungsform einer Siegelstation; und
Figur 14 eine weitere Ausführungsform einer Siegelstation.

### Wege zur Ausführung der Erfindung

Für das Vorgehen bei der Herstellung von Dosendeckeln mit Abreissfolie wird auf das zu den Figuren 1 bis 9 Gesagte verwiesen, was auch für die vorliegende Erfindung gilt. Diese wird ebenfalls anhand der Herstellung von Dosendeckeln beschrieben, ist aber grundsätzlich auch für andere Gegenstände mit Heisssiegelnähten verwendbar.

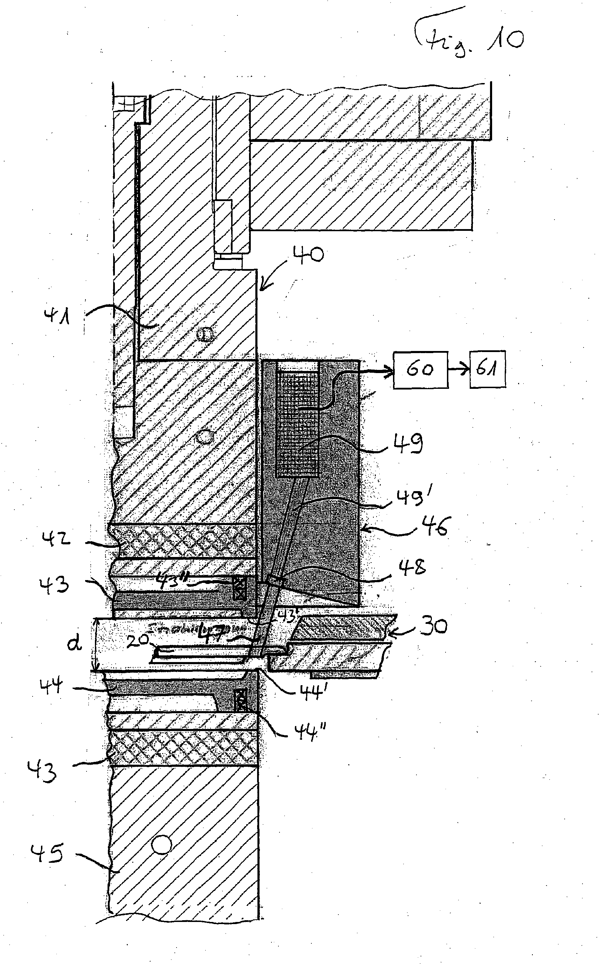

Die Figuren 10 bis 12 zeigen eine erste Ausführungsform einer Heisssiegelstation 40, die gemäss der vorliegenden Erfindung zur Herstellung von Gegenständen bzw. Deckeln 20 mit Heisssiegelnaht verwendet wird. In der Heisssiegelstation wird auf an sich bekannte Weise die Abreissfolie dichtend am Deckelring befestigt. Die Abreissfolie kann eine Mehrschichtfolie mit Kunststoffschichten und Aluminiumschichten sein, z.B. an der Deckelunterseite eine Schicht aus Polypropylen (PP) und eine folgende Schicht aus PET aufweisen, die von einer Aluminiumschicht gefolgt ist, welche an der Oberseite der Abreissfolie wieder mit einer PET-Schicht versehen ist. Eine allfällige Bedruckung ist dann unter dieser PET-Schicht angeordnet. Eine weitere Ausgestaltung der Abreissfolie kann eine deckelunterseitige bzw. füllgutseitige Heisssiegellackschicht gefolgt von der Aluminiumschicht und der deckeloberseitigen PET-Schicht sein. Auch weitere Ausgestaltungen sind dem Fachmann bekannt und können verwendet werden. Besonders die genannten Mehrschichtfolien sind auf einen stets korrekten Schweisstemperaturbereich angewiesen. Dieser ist je nach Folie unterschiedlich und ist vom Folienhersteller spezifiziert. Ist die Schweisstemperatur zu hoch, so kann sich eine Delaminierung einstellen, was auch möglich ist, wenn die Temperaturdifferenz zwischen der unteren Siegelscheibe und der oberen Siegelscheibe zu hoch ist. Bekannt ist auch, dass zu tiefe Schweisstemperaturen zur undichten Befestigung der Abreissfolie am Deckel führen kann. Gemäss der Erfindung wird daher so vorgegangen, dass bei der Deckelherstellung in der Siegelstation oder in einer nachfolgenden Station, in welcher die Siegelnaht von der Siegelung her noch heiss ist, ein Wärmeabbild des Gegenstandes bzw. von dessen Siegelnahtbereich aufgenommen wird. Fehlstellen sind auf einem solchen Wärmebild leicht zu erkennen, da sie gegenüber den korrekt versiegelten Stellen entweder zu heiss oder zu kalt sind, was sich im Wärmeabbild leicht feststellen lässt. Das Wärmeabbild wird dabei bei der Siegelstation aufgenommen, bei welcher die Siegelnaht fertiggestellt wird. Allenfalls kann eine vorgängige Siegelstation vorhanden sein, welche ein erstes Anheften der Abreissfolie bewirkt, die Siegelnaht aber noch nicht fertigstellt. An dieser Stelle ist es dann nicht vorgesehen, ein Wärmeabbild aufzunehmen. Anstelle der Aufnahme in der Siegelstation kann auch eine Aufnahme in einer nachfolgenden Station erfolgen, insbesondere in einer direkt in Förderrichtung nachfolgenden Station. Diese Station muss natürlich vom Gegenstand so schnell erreicht werden, dass die Temperatur der Siegelnaht noch so hoch ist, dass ein sinnvolles Wärmebild aufgenommen werden kann, ohne dass sich allfällige Temperaturdifferenzen von Fehlstellen bereits mit den benachbarten Gebieten der Siegelnaht ausgeglichen haben. Bevorzugt wird deshalb eine Aufnahme in der Siegelstation.

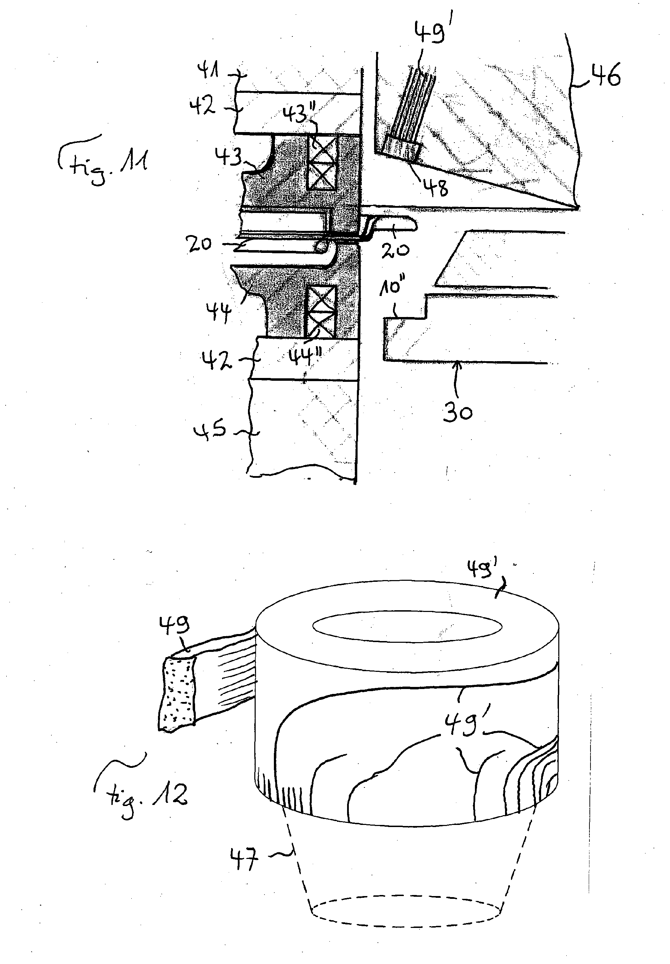

Die Figuren 10 bis 12 zeigen eine Ausführungsform für eine Aufnahme eines Wärmeabbildes in einer Siegelstation 40. Der Aufbau der eigentlichen Siegelstation ist dabei herkömmlich und dem Fachmann grundsätzlich bekannt und kann auch anders erfolgen als bei dem gezeigten Beispiel. Ein oberer Siegelkopf 41 trägt über eine Isolation 42 eine obere Siegelscheibe 43 mit Heizspiralen 43'', welche die eigentliche Siegelfläche 43' der Siegelscheibe 43 beheizen. Dies erfolgt über eine nicht dargestellte Temperatursteuerung oder -regelung mit der gewünschten Siegeltemperatur für die verwendete Abreissfolie. Im gezeigten Beispiel ist der obere Siegelkopf stationär. Der untere Siegelkopf 45 ist um den Zustellhub bzw. Öffnungshub d nach oben bzw. unten verfahrbar und trägt über eine weitere Isolation 43 die untere Siegelscheibe 44 mit den Heizspiralen 44'' und der eigentlichen Siegelfläche 44'. Die so erläuterte Siegelstation 40 ist in Figur 10 nur teilweise und ohne ihre Antriebsmittel dargestellt, da der Aufbau einer solchen Siegelstation dem Fachmann bekannt ist und hier nicht weiter erläutert werden muss. Ebenfalls nur teilweise dargestellt ist ein Deckel 20, der auf einer Transport- oder Fördereinrichtung 30 aufliegt, welche z.B. eine der erläuterten Fördereinrichtungen gemäss Figur 1 und insbesondere gemäss Figur 9 ist. In Figur 10 ist dabei die Siegelstation 40 mit dem oberen Siegelwerkzeug 41, 43 bzw. dem unteren Siegelwerkzeug 45, 44 in offener Stellung dargestellt, bei welcher der Deckelring 20 auf der Transporteinrichtung aufliegt. Dargestellt ist dabei weiter eine Stellung, bei welcher die Siegelung bereits erfolgt ist und mit der Abbildungseinrichtung ein Wärmeabbild der Siegelnaht aufgenommen wird, bevor der Gegenstand mittels der Transporteinrichtung 30 zur nächsten Station weiter gefördert wird. Figur 11 zeigt hingegen die geschlossene Stellung der Siegelwerkzeuge, bei welcher die eigentliche Siegelung der Siegelnaht erfolgt. Danach wird das Werkzeug wieder geöffnet, sodass sich die Stellung von Figur 10 ergibt. Anhand dieser wird nun eine Ausführungsform erläutert, bei welcher die Betrachtung der Siegelnaht durch ein wärmeempfindliches Betrachtungsmittel in der Siegelstation 40 erfolgt, so dass ein Wärmeabbild der Siegelnaht erzeugt bzw. aufgenommen wird.

Bei diesem Beispiel sind dazu Lichtleitermittel 49, 49' vorgesehen, durch welche hindurch eine Kamera 60 (nur schematisch als Funktionsblock dargestellt) bei geöffnetem Siegelwerkzeug den gesamten Heisssiegelnahtbereich des Deckels 20 betrachten kann. Dazu ist z.B. an der Siegelstation 40 ein Adapterteil 46 angeordnet, welches die Lichtleitermittel so aufnimmt, dass diese die Heisssiegelnaht betrachten. Dies ist in der Figur so dargestellt, dass ringförmig angeordnete, der jeweiligen Deckelform bzw. deren Siegelnahtform folgende Lichtleitermittel 49' vorgesehen sind, an deren einem Ende, allenfalls über eine Optik 48, die Wärmestrahlung von der Heisssiegelnaht aufnehmbar ist. Der Strahlengang zu dem Lichtleitermittel hin ist dabei mit 47 angedeutet. Die Wärmestrahlung bzw. Infrarotstrahlung, welche von der Siegelnaht ausgeht, wird somit über das Lichtleitermittel 49' entlang der gesamten Naht gleichzeitig aufgenommen. Weiter ist das Lichtleitermittel so ausgeführt, dass es anderends ein freies Ende 49 aufweist, an welchem die vom Lichtleiter aufgenommene Wärmestrahlung so angeordnet wieder abgegeben wird, dass sie von der Kamera 60 aufgenommen werden kann. Diese Kamera nimmt somit bei dieser Ausführungsform ein einzelnes Bild auf, welches die gesamte Heisssiegelnaht im Infrarotbereich aufgenommen darstellt. Dieses Bild wird dann in einer Auswerteinrichtung 61 ausgewertet, was noch erläutert wird. Lichtleitermittel sind grundsätzlich bekannt und müssen hier nicht genauer in ihrer Funktion erläutert werden. Ebenfalls sind Kameras bekannt, die Wärmebilder aufnehmen können bzw. die Bilder im Infrarotbereich aufnehmen.

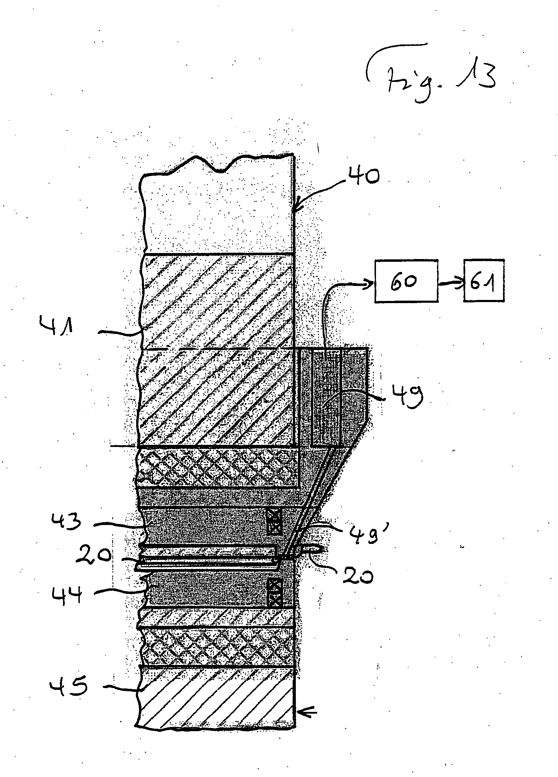

Die Ausführung des Lichtleitermittels 49, 49' kann natürlich auf vielfältige Weise erfolgen, welche für den Fachmann aufgrund der geschilderten Funktion wählbar ist. In Figur 12 ist eine Ausführungsform dargestellt, bei welcher das Lichtleitermittel durch eine Vielzahl von Glasfasern oder Glasfaserbündeln gebildet wird, welche in der dargestellten Weise ringförmig angeordnet sind, so dass der Eintritt der Infrarotstrahlung von der Heisssiegelnaht entlang deren ganzen Umfang in das Lichtleitermittel erfolgt und die Wärmestrahlung an einem Ort an die Kamera abgebbar ist. Je nach gewünschter Auflösung des Abbildes können einige hundert bis einige tausend Glasfasern entsprechend angeordnet sein. Das Lichtleitermittel kann aber auch aus einem einstückigen oder aus wenigen Stücken bestehenden geformten Lichtleiter ausgebildet sein. Wesentlich ist nur, dass die Siegelnaht bei geöffnetem Werkzeug betrachtbar ist und deren Abbild auf eine infrarotempfindliche Kamera oder einen anderen Sensor bringbar ist. Die Aufnahme bzw. die Erzeugung eines Abbildes des Heisssiegelbereiches kann aber auch so erfolgen, dass anstelle des gezeigten Lichtleitermittels eine Mehrzahl von Infrarotsensoren oder Infrarotthermometer um den Siegelbereich der Siegelstation 40 so verteilt sind, dass die Heisssiegelnaht bei geöffnetem Werkzeug direkt durch die Infrarotsensoren oder -thermometer betrachtbar ist, welche direkt ein elektrisches Signal abgeben, das dann ohne Kamera zur Auswertung 61 geführt werden kann. Solche Sensoren können z.B. an der mit 48 bezeichneten Stelle vorgesehen sein, mit welcher vorgängig eine allenfalls vorhandene Optik bezeichnet worden ist und sich ebenfalls ringförmig um die Siegelscheibe erstrecken. Derartige infrarotempfindliche Sensoren, allenfalls kombiniert mit einer Optik oder allenfalls mit einer an ihnen ohnehin vorgesehenen Optik oder Thermometer sind dem Fachmann bekannt und sind handelsüblich und brauchen hier nicht weiter erläutert zu werden. Diese werden dann mit einer elektrischen Speisung betrieben und deren Ausgangssignal wird über mehrere Leitungen an einen Anschluss am Adapter 46 geführt. Solche Sensoren können bei entsprechender Anzahl und Verteilung um die Siegelnaht herum das Wärmeabbild der gesamten Siegelnaht gleichzeitig aufnehmen. Die Auswertung kann aber dann auch sequentiell erfolgen, indem die Sensorsignale nacheinander abgefragt werden. Es ist auch möglich ein Aufnahmemittel, sei es mittels Lichtleitern oder mit Sensoren vorzusehen, durch welches nur ein Teil der Siegelnaht aufnehmbar ist und welches bei der Aufnahme entlang der Siegelnaht bewegt wird, wodurch diese in zeitlich aufeinanderfolgenden Wärmebildern dargestellt wird.

Nach der erläuterten Aufnahme des einen oder der mehreren Wärmebilder wird der Gegenstand 20 mittels der Transporteinrichtung weiter gefördert und der nächste Gegenstand gelangt in die Siegelstation 40 und wird dort nach Schliessen des Werkzeuges gemäss Figur 11 von der Transporteinrichtung abgehoben und heissgesiegelt, wonach erneut ein Öffnen des Werkzeuges erfolgt und die Aufnahme des Wärmebildes, wie vorstehend erläutert.

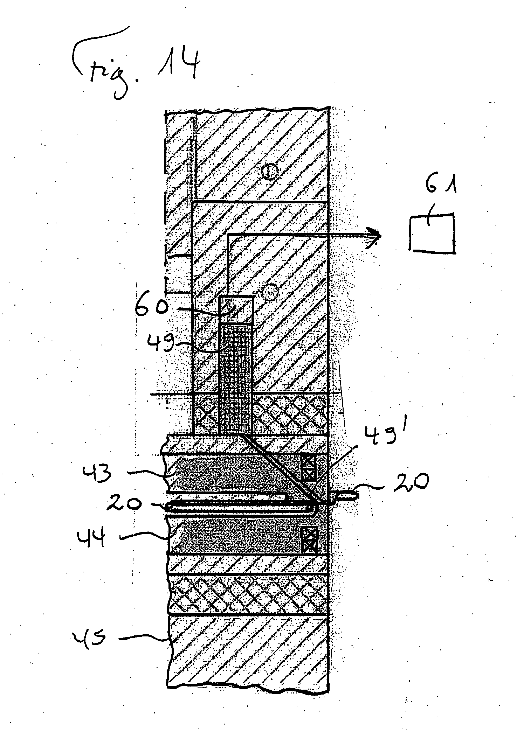

Die Figuren 13 und 14 zeigen weitere Ausführungsformen, bei welcher gleiche Bezugszeichen wiederum gleiche Elemente bezeichnen. Bei diesen Ausführungsformen kann die Aufnahme des Wärmeabbildes bei geschlossenem Siegelwerkzeug erfolgen, da das Lichtleitermittel 49' in die obere Siegelscheibe 43 hineingeführt ist und diese eine Vielzahl von Durchbrechungen aufweist, aus welchen das Lichtleitermittel in Form von einzelnen Lichtleitern, insbesondere Glasfasern oder Glasfaserbündeln, auch bei geschlossenem Werkzeug direkt auf die Siegelnaht blickt. Die Lichtleiter bzw. die Fasern oder Faserbündel, werden dann wieder zu einer Austrittsstelle 49 zusammengefasst, so dass eine Infrarotkamera eine Abbildung der Siegelnaht aufnehmen kann. Im Beispiel von Figur 13 sind die Faserbündel an dem der Kamera zugewandten Ende wiederum ausserhalb des eigentlichen Siegelkopfes angeordnet, bei der Ausführung von Figur 14 innerhalb des Siegelkopfes.

Bei allen dargestellten Ausführungsformen kann die Siegelkontur bzw. die Siegelnaht auf bekannte Weise rund, oval oder eckig sein, wie dies dem jeweiligen Gegenstand bzw. Deckel entspricht.

Steht das Wärmeabbild der Siegelnaht zur Verfügung, sei es als einzelnes Bild, als eine Abfolge von Bildern oder allenfalls lediglich als mit Infrarotsensoren aufgenommener Wärmeverlauf über die Siegelnahtlänge, so kann auf einfache Weise festgestellt werden, ob die Siegelnaht korrekt versiegelt worden ist. Dies kann durch Auswertung des Bildes bzw. der Bilder oder des Temperaturverlaufes erfolgen, womit feststellbar ist, ob die Temperatur sich in einem vorgegebenen Sollbereich befindet. Dazu wird das Bild durch Bildverarbeitung ausgewertet und z.B. zeilenweise ausgewertet. Für die Auswertung 61 kann dabei der Rechner und ein marktübliches Bildverarbeitungsprogramm verwendet werden. Sowohl zu heisse Stellen als zu kalte Stellen, womit gemeint ist, dass diese Stellen ausserhalb des vorgegebenen, für die jeweilige Abreissfolie bzw. das Siegelnahtmaterial zulässigen Temperaturbereich zeigen fehlerhafte Gegenstände bzw. Deckel an. Im Bild werden solche Stellen als gegenüber den Stellen mit korrekter Temperatur zu helle bzw. zu dunkle Stellen gezeigt, was mit einer einfachen Bildauswertung feststellbar ist. Solches ist dem Fachmann bekannt und wird hier nicht weiter ausgeführt. Deckel, welche bei der Herstellung als fehlerhaft erkannt werden, werden aussortiert. Dies erfolgt z.B. wie bei der Figur 1 dargestellt durch entsprechende verschiedene Auslässe für die Deckel am Ende der Herstellung. Auch ein Ausscheiden direkt nach der Siegelstation ist natürlich möglich. Neben einer einfachen Temperaturbetrachtung anhand des Bildes oder des Temperaturverlaufes mit einem oberen und unteren Grenzwert können natürlich insbesondere anhand eines Bildes auch genauere Auswertungen und insbesondere der Versuch einer Fehlerursachenerkennung erfolgen. Insbesondere kann festgestellt werden, ob Fehler durch zu heisse oder zu tiefe Temperatur auftreten und ob diese stets an derselben Stelle auftreten, wie gross die Fehlstellen sind usw.

## Patentansprüche

1. Verfahren zur Herstellung von Gegenständen (20), insbesondere Deckeln mit einer Abreissfolie, mit einer Siegelnaht, die bei der Herstellung in einer Siegelstation (40) gebildet wird, **dadurch gekennzeichnet, dass** in der Siegelstation oder in einer an die Siegelstation anschliessenden Station ein Wärmeabbild der Siegelnaht aufgenommen und ausgewertet wird.

2. Verfahren nach Anspruch 1, **dadurch gekennzeichnet, dass** das Wärmeabbild bei geöffneten Siegelwerkzeugen aufgenommen wird.

3. Verfahren nach Anspruch 1, **dadurch gekennzeichnet, dass** das Wärmeabbild bei geschlossenen Siegelwerkzeugen durch die eine Siegelscheibe hindurch aufgenommen wird.

4. Verfahren nach einem der Ansprüche 1 bis 3, **dadurch gekennzeichnet, dass** die gesamte Siegelnaht mit einem Bild erfasst wird.

5. Verfahren nach einem der Ansprüche 1 bis 3, **dadurch gekennzeichnet, dass** die Siegelnaht mit mehreren Teilbildern erfasst wird.

6. Verfahren nach Anspruch 5, **dadurch gekennzeichnet, dass** die Teilbilder gleichzeitig mit stationären Mitteln aufgenommen werden und/oder, dass die Teilbilder nacheinander mit bewegten Aufnahmemitteln aufgenommen werden.

7. Verfahren nach einem der Ansprüche 1 bis 6, **dadurch gekennzeichnet, dass** das Abbild über eine Vielzahl von Glasfasern erfasst wird, welche eingangsseitig der Form der Siegelnaht folgend angeordnet sind und ausgangseitig zusammengefasst sind, um das Bild mittels einer infrarotempfindlichen Kamera aufzunehmen.

8. Vorrichtung zur Herstellung von mit Siegelnähten versehenen Gegenständen, insbesondere von Deckeln mit Abreissfolie, umfassend eine Fördereinrichtung für die Gegenstände sowie eine Mehrzahl von entlang der Fördereinrichtung angeordneter Bearbeitungsstationen umfassend eine Siegelstation (40), **dadurch gekennzeichnet, dass** eine Einrichtung (46) zur Aufnahme eines Wärmeabbildes der Siegelnaht des Gegenstandes vorgesehen ist, welche in oder an der Siegelstation oder in oder an einer nachfolgenden Station vorgesehen ist.

9. Vorrichtung nach Anspruch 8, **dadurch gekennzeichnet, dass** die Einrichtung zur Aufnahme eines Wärmeabbildes bei offenem Siegelwerkzeug der Siegelstation ausgebildet ist.

10. Vorrichtung nach Anspruch 8, **dadurch gekennzeichnet, dass** die Einrichtung zur Aufnahme eines Wärmeabbildes bei geschlossenem Siegelwerkzeug ausgebildet ist und dazu eine Vielzahl von durch die eine Siegelscheibe des Siegelwerkzeuges hindurch geführte Glasfasern aufweist.

11. Vorrichtung nach einem der Ansprüche 8 bis 10, **dadurch gekennzeichnet, dass** die Fördereinrichtung parallel verlaufende endlose Zahnriemen aufweist:
